# EUROPÄISCHE PATENTSCHRIFT

(11) **EP 2 151 207 B1**
(45) Veröffentlichungstag und Bekanntmachung des Hinweises auf die Patenterteilung: **07.03.2012**
(21) Anmeldenummer: 09014615.0
(22) Anmeldetag: 13.04.2005
(51) Int. Cl.: A61B 17/70, A61B 17/68, A61B 17/86

(54) **Elastisches Element zur Verwendung in einer Stabilisierungseinrichtung für Knochen und Herstellungsverfahren für ein solches elastisches Element**
Elastic element for use in a stabilising device for bones and method of fabrication of such an elastic element
Élément élastique pour l'usage dans un dispositif de stabilisation pour des os et la méthode de fabrication d'un tel élément élastique

(30) Priorität: 16.04.2004 DE 102004018621; 16.04.2004 US 563241 P
(43) Veröffentlichungstag der Anmeldung: 10.02.2010
(62) Teilanmeldung aus: 05008060.5
(73) Patentinhaber: Biedermann Motech GmbH, 78054 VS-Schwenningen (DE)
(72) Erfinder: Harms, Jürgen, 76227 Karlsruhe (DE); Biedermann, Lutz, 78048 VS-Villingen (DE); Matthis, Wilfried, 79367 Weisweil (DE)
(74) Vertreter: Prüfer & Partner GbR European Patent Attorneys

(56) Entgegenhaltungen:
- EP-A2- 0 529 275
- WO-A-2004/105577
- US-A- 5 500 122
- US-A1- 2004 073 218
- US-B1- 6 197 065
- US-B1- 6 656 184

## Beschreibung

Die Erfindung betrifft ein elastisches Element für die Verwendung in der Wirbelsäulen- oder Unfallchirurgie, ein Knochenverankerungselement, ein stabförmiges Element und eine Stabilisierungseinrichtung jeweils mit einem solchen elastischen Element, sowie ein Herstellungsverfahren für ein solches elastisches Element.

Zur Fixierung von Knochenfrakturen oder zur Stabilisierung der Wirbelsäule sind Fixations- und Stabilisierungseinrichtungen bekannt, die aus wenigstens zwei im Knochen bzw. Wirbel verankerten und über eine Platte oder über einem Stab verbundenen Knochenschrauben bestehen. Derartige starre Systeme erlauben keine Bewegung der relativ zueinander fixierten Knochenteile oder Wirbel.

Für bestimmte Indikationen ist jedoch eine dynamische Stabilisierung wünschenswert, bei der die zu stabilisierenden Knochenteile und Wirbel eine kontrollierte begrenzte Bewegung zu einander ausführen können. Eine Möglichkeit für die Realisierung der dynamischen Stabilisierungseinrichtung besteht in der Verwendung eines elastischen Elementes anstelle eines die Knochenverankerungselemente verbindenden starren Stabes.

Aus der US 2003/0109880 A1 ist eine dynamische Stabilisierungseinrichtung für Wirbel bekannt, die eine erste und eine zweite im Wirbel zu verankernde Schraube jeweils mit einem Aufnahmeteil zum Einlegen einer die Schrauben verbindenden Feder und eine solche Feder umfaßt. Die Feder selbst ist als Ganzes in Form einer Schraubenfeder mit dicht benachbarten Windungen nach Art einer Zugfeder ausgebildet und wird über Klemmschrauben in den Aufnahmeteilen fixiert. Es besteht hierbei jedoch die Gefahr, dass die Feder auf Grund ihrer Elastizität dem Druck der Klemmschraube ausweicht und somit die Fixierung zwischen der Knochenschraube und der Feder gelockert wird. Ein weiterer Nachteil der Vorrichtung besteht darin, dass die Elastizität der Feder bei ansonsten gleichen Federeigenschaften von der Länge der Feder abhängt. Ferner ist die Biegesteifigkeit der Feder insbesondere bei kurzen Baulängen relativ gering.

Aus der US 6,162,223 ist eine Fixationseinrichtung für ein Gelenk, z.B. für ein Handgelenk oder ein Kniegelenk, bekannt, bei der ein an seinen Enden mit Knochenverankerungselementen verbundener Fixationsstab zweiteilig ausgebildet ist, wobei die zwei Teile des Fixationsstabs über ein flexibles Kupplungsteil miteinander verbunden sind und wobei die Fixationsstäbe um das Kupplungsteil außerhalb des Körpers angebracht sind. Die beiden Teile des Fixationsstabs sind mit dem Kupplungsteil nicht fest verbunden, sondern können sich entlang einer Bohrung in dem Kupplungsteil frei bewegen. Der Durchmesser des Kupplungsteil ist auf Grund der Art der Verbindung mit dem zweiteiligen Fixationsstab immer größer als der Durchmesser des Fixationsstabs. Diese bekannte Fixationseinrichtung ist auf Grund ihres komplizierten und voluminösen Aufbaus zum körperinternen Einsatz an der Wirbelsäule oder anderen Knochen nicht geeignet. Insbesondere die Realisierung eines solchen flexiblen Kupplungsteils mit hoher Biegesteifigkeit erfordert ein großes Bauvolumen.

Es ist Aufgabe der Erfindung, ein elastisches Element bereit zu stellen, das eine hohe Biegesteifigkeit bei geringer Baulänge hat, sowie leicht zu handhaben ist bei gleichzeitig hoher Sicherheit im Einsatz, und welches mit anderen Elementen in möglichst vielfältiger Weise zu einer dynamischen Stabilisierungseinrichtung für Wirbel oder Knochen kombiniert werden kann. Ferner ist es Aufgabe der Erfindung, ein Herstellungsverfahren für das elastische Element bereit zu stellen.

Die Aufgabe wird gelöst durch ein elastisches Element nach dem Patentanspruch 1 und ein Verfahren zum Herstellen eines solchen elastischen Elements nach dem Patentanspruch 11.

Weiterbildungen der Erfindung sind in den Unteransprüchen angegeben.

Die Erfindung weist den Vorteil auf, dass ein elastisches Element zugleich kompakt und mit hoher Biegesteifigkeit ausgebildet ist. Dies ist insbesondere für Anwendungen an der Wirbelsäule und dort insbesondere an der Halswirbelsäule von Bedeutung, wo der zu Verfügung stehende Platz im Vergleich zu Anwendungen an der Lendenwirbelsäule deutlich geringer ist. Ferner weist die Erfindung den Vorteil auf, dass ein elastisches Element wahlweise mit starren stabförmigen Elementen verschiedener Länge zu einem elastischen stabförmigen Element kombinierbar ist oder mit verschiedenen Schäften und/oder Köpfen zu einer Knochenschraube mit elastischen Eigenschaften kombinierbar ist. Das elastische stabförmige Element bzw. die Knochenschraube weisen dann in Abhängigkeit von dem verwendetem elastischen Element vorgegebene elastische Eigenschaften wie eine bestimmte Kompressions- und Extensionsfähigkeit in axialer Richtung, sowie eine bestimmte Biege- und Torsionssteifigkeit auf.

Insbesondere kann das elastisches Element mit stabförmigen Bauteilen verschiedener Dicke oder mit in der Wirbelsäulen-und/oder Unfallchirurgie zu verwendenden Platten unterschiedlicher Form und Länge verbunden werden.

Weitere Merkmale und Zweckmäßigkeiten ergeben sich aus der Beschreibung von Ausführungsbeispielen anhand der Figuren.

Von den Figuren zeigen:
- Fig. 1: ein elastisches Element mit einer Doppelschraubenfeder ;
- Fig. 2: eine schematische Darstellung der Doppelschraubenfe- der des elastischen Elements aus Fig. 1 einmal als Ganzes (Fig. 2a) und in Explosionsdarstellung (Fig. 2b);
- Fig. 3: ein elastisches Element mit einer Doppelschraubenfe der ;
- Fig. 4a, 4b: ein elastisches Element mit einer Doppelschrau- benfeder nach der Ausführungsform der Er- findung, einmal als Ganzes (Fig. 4a) und einmal in Explosionsdarstellung (Fig. 4b);
- Fig. 5a: ein elastisches Element mit einer Doppelschraubenfe- der der Erfin- dung;
- Fig. 5b: eine Schnittdarstellung des elastischen Elements aus Fig. 5a;
- Fig. 6: eine Schnittdarstellung eines elastischen Elements mit einer Doppelschraubenfeder der Erfindung;
- Fig. 7a: ein elastisches Element mit einer Doppelschraubenfe der ;
- Fig. 7b: eine Schnittdarstellung des elastischen Elements aus Fig. 7a;
- Fig. 8a: ein stabförmiges Element mit dem elastischen Element;
- Fig. 8b: eine teilgeschnittene Explosionsdarstellung einer Polyxialknochenschraube mit dem elastischen Element;
- Fig. 8c: eine teilgeschnittene Darstellung einer Monoaxial- schraube mit dem elastischen Ele- ment;
- Fig. 8d: eine Ansicht von oben auf einen aus einer Platte, dem elastischen Element und einen Stababschnitt bestehenden Teil einer Stabilisie- rungsvorrichtung für Knochen oder Wirbel, sowie ei- nen Querschnitt durch die Platte;
- Fig. 9: eine Anwendung des elastischen E- lementes in einer Stabilisierungseinrichtung für die Wirbelsäule;
- Fig. 10a-c: Schritte einer Ausführungsform eines Verfahrens zur Herstellung eines elastischen Elementes; und
- Fig. 11: ein elastisches Element mit einem halbkreisförmigen Auslauf an beiden Enden der Dop- pelschraubenfeder.

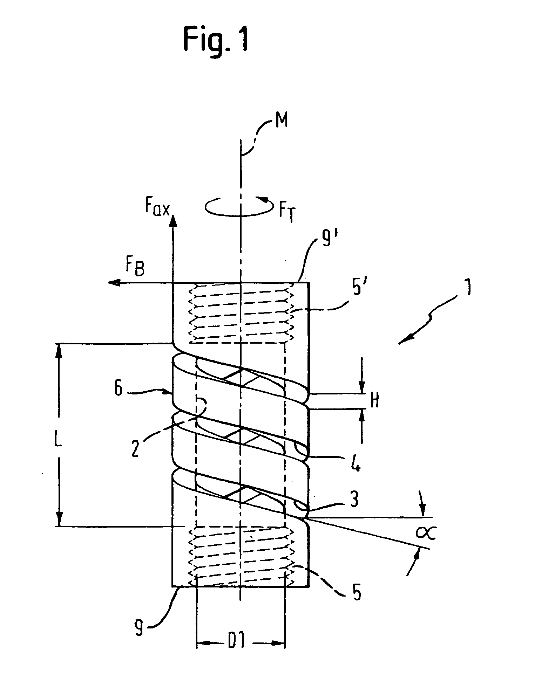

In Fig. 1 ist ein elastisches Element 1 dargestellt.

Das elastische Element 1 ist als hohlzylindrisches Element mit einer durchgehenden koaxialen Bohrung 2 und einer spiralförmig mit einer vorbestimmten Steigung und über eine vorbestimmte Länge in Richtung der Zylinderachse in der Wandung verlaufenden ersten Ausnehmung 3, die in radialer Richtung in die Bohrung 2 mündet, ausgebildet. Weiter ist spiralförmig mit der gleichen Steigung wie die erste Ausnehmung 3 über die gleiche Länge in Richtung der Zylinderachse M in der Wandung zwischen den Windungen der ersten Ausnehmung 3 eine zweite Ausnehmung 4 vorgesehen, die in radialer Richtung in die Bohrung 2 mündet. Dadurch ist eine Doppelschraubenfeder aus zwei Schraubenfedern gebildet, wobei die Windungen der einen Schraubenfeder zwischen den Windungen der zweiten Schraubenfeder verlaufen. Bevorzugt sind die Windungen der einen Schraubenfeder relativ zu den Windungen der anderen Schraubenfeder um ihre gemeinsame Mittenachse um 180° gedreht, sodass sich die erste und die zweite Ausnehmung genau gegenüberliegen.

Die Länge L der spiralförmigen Ausnehmungen 3, 4 in Richtung der Zylinderachse, die Höhe H der Ausnehmungen, die Steigung α der Schraubenlinien, entlang denen die Ausnehmungen 3, 4 ausgebildet sind, und der Durchmesser D1 der koaxialen Bohrung 2 sind so gewählt, dass eine gewünschte Steifigkeit des elastischen Elements gegenüber axialen Kräften Fₐₓ, Biegekräften F_{B} und Torsionskräften F_{T}, die auf das elastische Element wirken, gegeben ist. Angrenzend an seine beiden freien Enden weist das elastische Element 1 jeweils einen Abschnitt mit einem sich über eine vorbestimmte Länge erstreckenden Innengewinde 5, 5' auf. Die Innengewindeabschnitte überlappen in axialer Richtung nicht mit dem Abschnitt, in dem die Ausnehmungen in der Wandung ausgebildet sind. Der Außendurchmesser des elastischen Elements 1 ist der jeweiligen Anwendung entsprechend gewählt. Das elastische Element ist aus einem körperfreundlichen Material wie z.B. Titan ausgebildet.

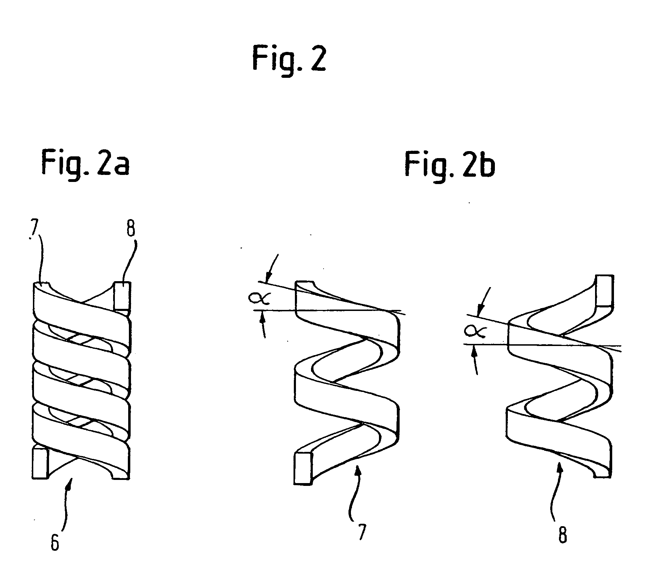

Fig. 2 veranschaulicht den Aufbau einer Doppelschraubenfeder 6 wie sie durch die Ausnehmungen 3, 4 in dem elastischen Element in Fig. 1 gebildet wird.

Die Doppelschraubenfeder 6 setzt sich zusammen aus zwei .Schraubenfedern 7 und 8. Die beiden Schraubenfedern 7 und 8 sind identisch ausgebildet und besitzen insbesondere eine identische Steigung α, jedoch ist die erste Schraubenfeder 7 in der Doppelschraubenfeder 6 gegenüber der zweiten Schraubenfeder 8 um ihre gemeinsame Mittenachse um 180° gedreht. Die Windungen der ersten Schraubenfeder 7 verlaufen bei der Doppelschraubenfeder 6 daher in der Mitte zwischen den Windungen der zweiten Schraubenfeder 8 und umgekehrt.

Bei einer vorgegebenen Baulänge eines elastischen Elementes ist die Steigung der Schraubenfeder dadurch begrenzt, dass mindestens eine ganze Windung vorhanden sein muss, um gute Elastizitätseigenschaften zu erhalten. Bei einer Doppelschraubenfeder ist für jeden der Schraubenfedern weniger als eine ganze Windung notwendig, um gute Elastizitätseigenschaften zu erhalten. Daher kann bei gleicher Baulänge die Steigung der Schraubenfeder im Vergleich zu einer Einfachschraubenfeder erhöht werden. Eine Erhöhung der Steigung der Schraubenfeder ergibt bei gleicher Baulänge und gleichen sonstigen Eigenschaften eine Erhöhung der Biegesteifigkeit. Daher ist es mit einer Doppelschraubenfeder gegenüber einer Einfachschraubenfeder möglich bei gleichen Abmessungen eine höhere Biegesteifigkeit zu erreichen.

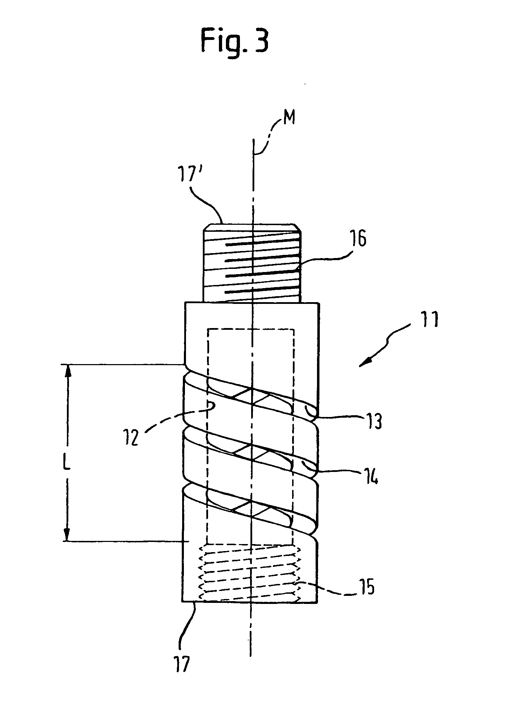

In Fig. 3 ist ein elastisches Element 11 dargestellt.

Dieses elastische Element 11 unterscheidet sich von dem elastischen Element 1 dadurch, dass anstelle einer sich von dem ersten bis zu dem zweiten Ende 17' des elastischen Elementes erstreckenden Bohrung 2 eine an das erste Ende 17 des elastischen Elements angrenzende Sackbohrung 12 vorgesehen ist. Die Sackbohrung erstreckt sich dabei über die gesamte Länge L der Doppelschraubenfeder, die wie bei dem Element 1 durch eine erste und eine zweite Ausnehmung 13 und 14 in der Wandung eines hohlzylindrischen Abschnittes gebildet wird. An das erste Ende 17 angrenzend ist in der Sackbohrung ein Innengewinde 15 vorgesehen. An dem dem ersten Ende 17 gegenüberliegenden zweiten Ende 17' weist das elastische Element einen zylinderförmigen Ansatz 16 mit einem Außengewinde auf.

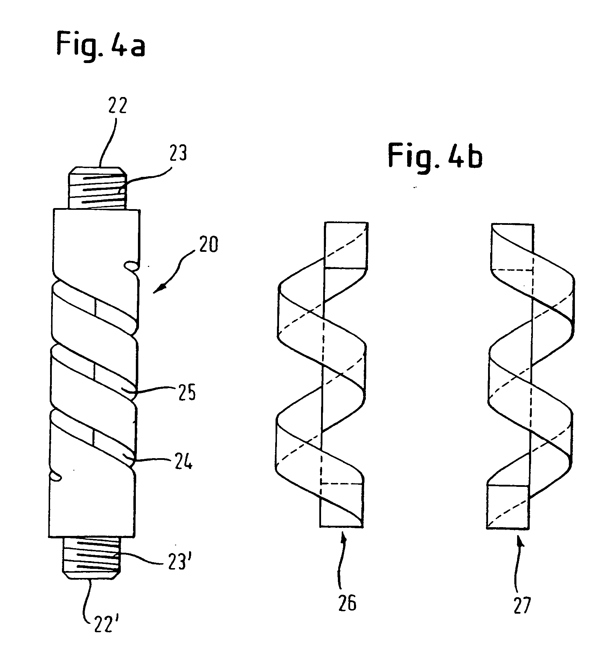

In Fig. 4 ist ein elastisches Element nach der Ausführungsform der Erfindung dargestellt.

Das elastische Element 20 unterscheidet sich dadurch von den anderen Elementen dass keine Bohrung koaxial zur Mittenachse M des elastischen Elementes 20 vorgesehen ist. Des weiteren ist sowohl an das erste Ende 22, als auch an das zweite Ende 22' angrenzend ein zylindrischer Ansatz 23, 23' mit einem Außengewinde vorgesehen. Wie bei den zuvor beschriebenen Ausführungsformen werden durch zwei Ausnehmungen 24, 25 zwei Schraubenfedern 26, 27 gebildet.

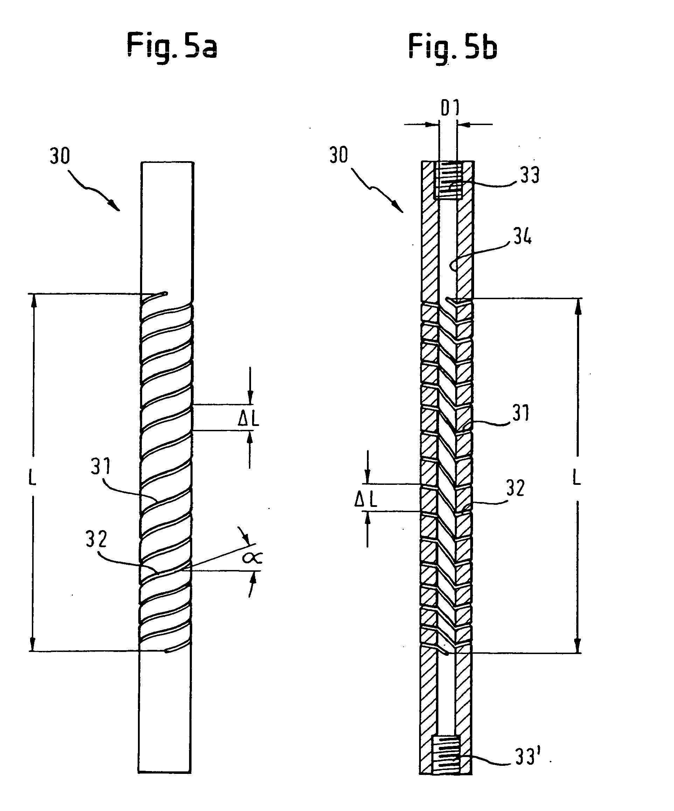

In Fig. 5a ist ein elastisches Element dargestellt. Fig. 5b ist eine Schnittdarstellung des elastischen Elements aus Fig. 5a.

Das elastische Element 30 unterscheidet sich von dem elastischen Element 1 dadurch, dass die Steigung α der die Doppelschraubenfeder bildenden Ausnehmungen 31, 32 nicht konstant ist, sondern über die Länge L der Doppelschraubenfeder des elastischen Elements 30 variiert. Dabei variiert die Steigung α derart, dass der Abstand ΔL der Ausnehmungen 31, 32 von den freien Enden des elastischen Elements 30 aus gesehen zur Mitte hin zunimmt. Dementsprechend variiert auch die Biegesteifigkeit des elastische Elements 30, die mit zunehmendem Abstand ΔL der Ausnehmungen 31, 32 zunimmt. Über die Steigung der Ausnehmungen entlang der Mittenachse des elastischen Elements kann somit gezielt eine bestimmte ortsabhängige Biegesteifigkeit eingestellt werden.

Wie das Element 1 weist das elastische Element 30 angrenzend an seine beiden freien Enden jeweils einen Abschnitt mit einem sich über eine vorbestimmte Länge erstreckenden Innengewinde 33, 33' und eine durchgehende koaxiale Bohrung 34 mit einem Innendurchmesser D1 auf.

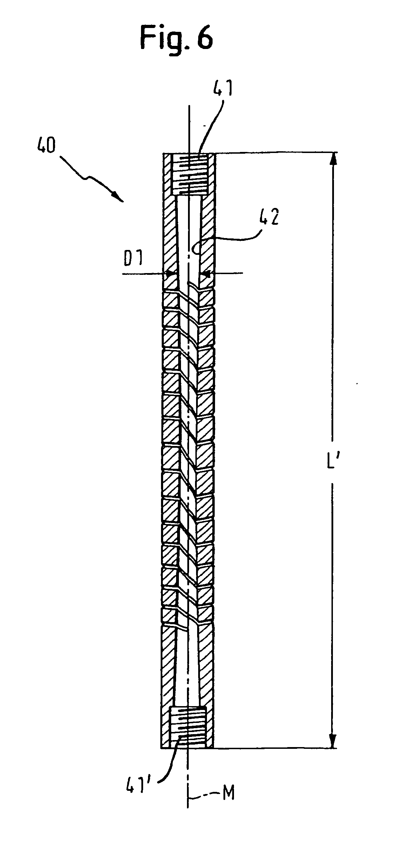

Fig. 6 ist eine Schnittdarstellung eines elastischen Elements.

Das elastische Element 40 unterscheidet sich von dem elastischen Element 30 dadurch, dass der Innendurchmesser D1 der durchgehenden koaxialen Bohrung 42 nicht konstant ist, sondern über die Länge L' des elastischen Elements 40 variiert. Der Innendurchmesser D1 der Bohrung 42 variiert dabei derart, dass er von den freien Enden aus gesehen zur Mitte des elastischen Elements 40 hin abnimmt. Dementsprechend variiert auch die Biegesteifigkeit des elastischen Elements 40, die mit abnehmendem Innendurchmesser D1 zunimmt. Über den Innendurchmesser der koaxialen Bohrung kann somit gezielt eine bestimmte ortsabhängige Biegesteifigkeit des elastischen Elements 40 eingestellt werden.

Wie das Element 30 weist das elastische Element 40 angrenzend an seine beiden freien Enden jeweils einen Abschnitt mit einem sich über eine vorbestimmte Länge erstreckenden Innengewinde 41, 41'.

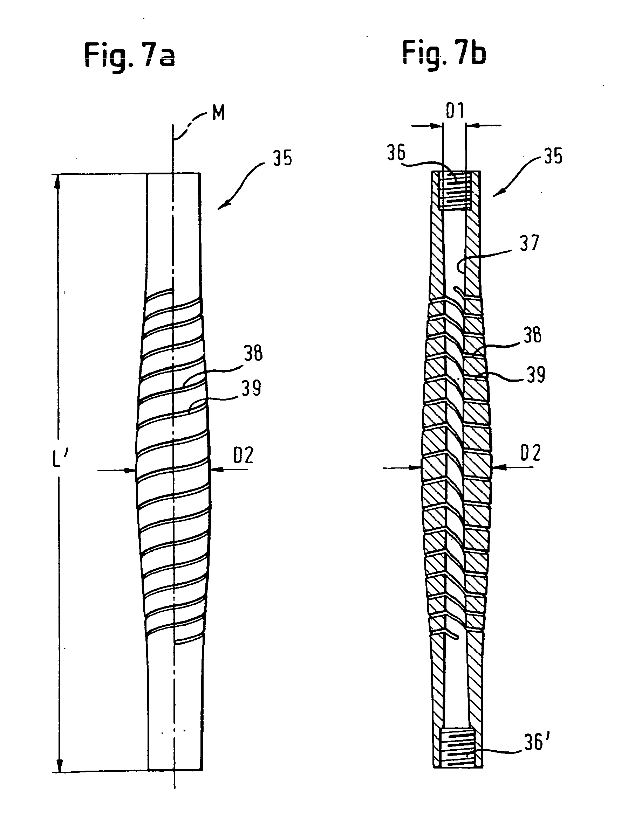

In Fig. 7a ist ein elastisches Element dargestellt. Fig. 7b ist eine Schnittdarstellung des elastischen Elements aus Fig. 7a.

Das elastische Element 35 unterscheidet sich von dem Element 40 dadurch, dass der Außendurchmesser D2 des elastischen Elements 35 nicht konstant ist, sondern über die Länge L' de des elastischen Elements 35 variiert. Dabei variiert der Außendurchmesser D2 derart, dass er von den freien Enden aus gesehen zur Mitte des elastischen Elements 35 hin zunimmt. Dementsprechend variiert auch die Biegesteifigkeit des elastischen Elements 35, die mit zunehmendem Außendurchmesser des elastischen Elements zunimmt. Über den Außendurchmesser des elastischen Elements kann somit gezielt eine bestimmte ortsabhängige Biegesteifigkeit eingestellt werden.

Wie das Element 30 weist das elastische Element 35 angrenzend an seine beiden freien Enden jeweils einen Abschnitt mit einem sich über eine vorbestimmte Länge erstreckenden Innengewinde 36, 36', eine durchgehende koaxiale Bohrung 37 mit einem Innendurchmesser D1 sowie zwei Ausnehmungen 38 und 39 in der Form einer Schraubenfeder auf, durch die eine Doppelschraubenfeder gebildet wird.

Abwandlungen des elastischen Elementes von den zuvor beschriebenen Ausführungsformen sind möglich.

So kann das elastische Element mehr als zwei Schraubenfedern beinhalten, wobei die Windungen einer Schraubenfeder jeweils zwischen den Windungen der anderen Schraubenfedern verlaufen.

In einer weiteren Abwandlung des Elements 11 ist anstelle der Sackbohrung 12 eine sich über die gesamte Länge des elastischen Elementes erstreckende Bohrung vorgesehen, deren Durchmesser kleiner als der Außendurchmesser des zylindrischen Ansatzes 16 ist.

Die Elemente 30, 35, 40 wurde jeweils so beschrieben, dass die Biegesteifigkeit des elastische Elements über die Länge L der Doppelschraubenfeder von den freien Enden aus gesehen zur Mitte des elastischen Elements hin zunimmt. Jedoch kann durch entsprechende Gestaltung der Steigung α der beiden Ausnehmungen, des Außendurchmessers D2 des elastischen Elements und des Innendurchmesser D1 der koaxialen Bohrung die Biegesteifigkeit mit einer beliebigen andere Ortsabhängigkeit über die Länge L der Doppelschraubenfeder bzw. die Lange L' des elastischen Elements eingestellt werden.

Alle Elementen wurden so beschrieben, dass das elastische Element die Form eines Zylinders bzw. Hohlzylinders aufweist, jedoch kann die äußere Form auch von der exakten Form eines Zylinders abweichen und z.B. eine ovale Querschnittsfläche haben oder tailliert ausgebildet sein. Das elastische Element hat damit eine richtungsabhängige Biegeelastizität:

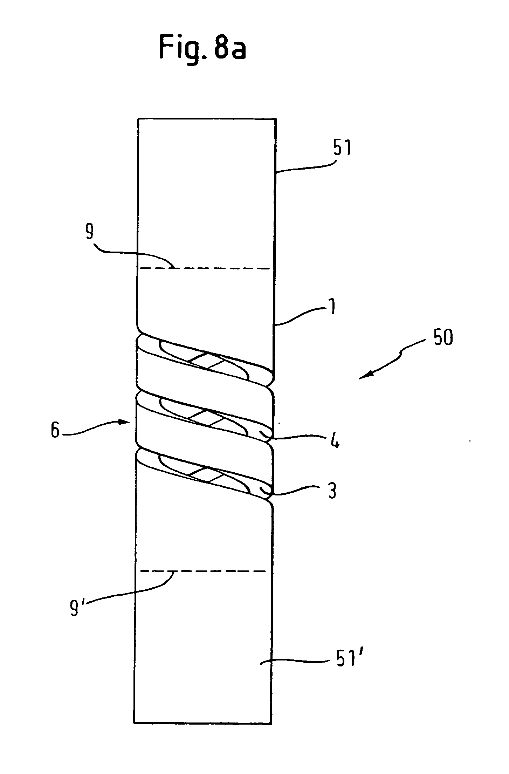

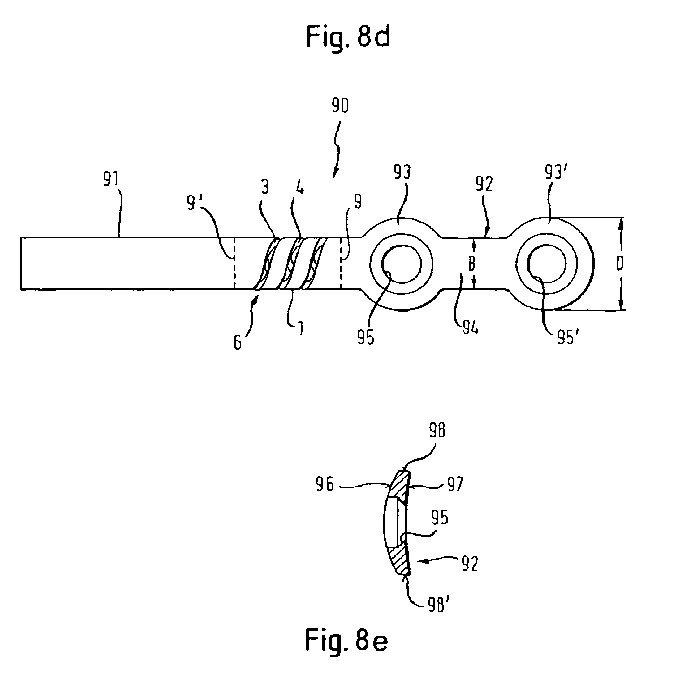

In einem ersten, in Fig. 8a gezeigten Anwendungsbeispiel ist das elastische Element 51 Bestandteil eines elastischen stabförmigen Elements 50. Das elastische stabförmige Element 50 besteht aus dem Federelement 1 und zwei zylindrischen Stabschnitten 51, 51', die an ihrem Ende jeweils einen nicht dargestellten zylindrischen Ansatz mit einem Außengewinde aufweisen, das jeweils mit einem Innengewinde 5 bzw. 5' des Federelements 1 zusammenwirkt. Die Stababschnitte 51, 51' und das Federelement 1 weisen in diesem Ausführungsbeispiel im wesentlichen denselben Außendurchmesser auf. Die Länge der Stababschnitte 51, 51' und des Federelements 1 sind unabhängig voneinander im Hinblick auf eine gewünschte Anwendung wählbar. Das elastische stabförmige Element 50 dient beispielsweise zur Verbindung von Pedikelschrauben an der Wirbelsäule. Das so gebildete stabförmige Element 30 nimmt durch die elastischen Eigenschaften des Federelementes 1 in vorbestimmten Umfang Kompressions-, Extensions-, Biege- und Torsionskräfte auf.

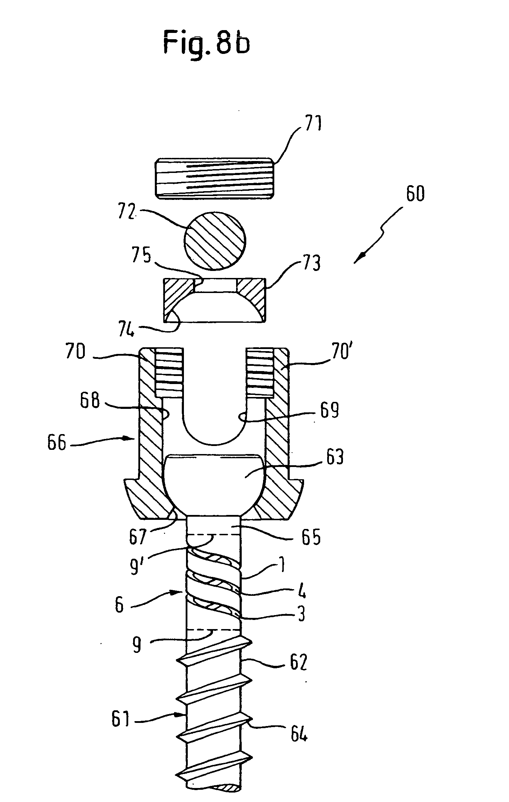

Fig. 8b zeigt ein zweites Anwendungsbeispiel des elastischen Elementes 1. Das elastischen Element 1 ist hier Bestandteil eines Knochenverankerungselements, das als PolyaxialKnochenschraube 60 ausgebildet ist.

Die Polyaxialknochenschraube 60 weist ein Schraubenelement 61 auf, welches aus dem elastischen Element 1, einem Gewindeschaft 62 in diesem Ausführungsbeispiel mit einer nicht dargestellten Spitze und einem Schraubenkopf 63 besteht.Der Gewindeschaft 62 weist ein Knochengewinde 64 zum Einschrauben in den Knochen und einen nicht dargestellten zylinderförmigen Ansatz mit einem Außengewinde auf, das mit dem Innengewinde 5 des Federelementes 1 zusammenwirkt. Der Schraubenkopf 63 weist einen zylinderförmigen Abschnitt 65 und daran angrenzend wie der Gewindeschaft 62 einen nicht dargestellten zylinderförmigen Ansatz mit einem Außengewinde auf, das mit dem Innengewinde 5' des Federelementes 1 zusammenwirkt.

Das Schraubenelement 61 ist in einem Aufnahmeteil 66 in unbelastetem Zustand schwenkbar gehalten. Das Aufnahmeteil 66 ist im wesentlichen zylindrisch ausgebildet und weist an seinem einen Ende eine axialsymmetrisch ausgerichtete erste Bohrung 67 auf, deren Durchmesser größer als der des Gewindeschafts 62 und kleiner als der des Schraubenkopfs 63 ist. Ferner weist das Aufnahmeteil 66 eine koaxiale zweite Bohrung 68 auf, die auf dem der ersten Bohrung 67 gegenüberliegenden Ende offen ist und deren Durchmesser so groß ist, dass das Schraubenelement 61 durch das offene Ende mit dem Gewindeschaft 62 durch die erste Bohrung 67 hindurchführbar ist, bis der Schraubenkopf 63 am Rand der ersten Bohrung 67 anliegt. Das Aufnahmeteil 66 weist eine sich vom freien Ende in Richtung der ersten Bohrung 67 erstreckende U-förmige Ausnehmung 69 auf, durch die zwei freie Schenkel 70, 70' gebildet sind. In einem Bereich angrenzend an ihr freies Ende weisen die Schenkel 70, 70' ein Innengewinde auf, welches mit einem entsprechenden Außengewinde einer Innenschraube 71 zum Fixieren eines Stabs 72 zusammenwirkt.

Es ist ferner ein Druckelement 73 zum Fixieren des Schraubenkopfs 63 in dem Aufnahmeteil 66 vorgesehen, das so ausgebildet ist, dass es an seiner dem Schraubenkopf 63 zugewandten Seite eine sphärische Ausnehmung 74 aufweist, deren Radius im wesentlichen gleich dem Radius des kugelsegmentförmigen Abschnitts des Schraubenkopfes 63 ist. Der Außendurchmesser des Druckelements 73 ist so gewählt, dass es in dem Aufnahmeteil 66 zu dem Schraubenkopf 63 hin verschiebbar ist. Das Druckelement 73 weist ferner eine koaxiale Bohrung 75 für den Zugriff auf eine nicht dargestellte Ausnehmung in dem Schraubenkopf 63 zum Ineingriffbringen mit einem Einschraubwerkzeug auf.

Im Betrieb wird in das Innengewinde 5 des elastischen Elementes 1 der Gewindeschaft 62 mit seinem nicht dargestellten zylinderförmigen Ansatz und in das Innengewinde 5' der Schraubenkopf 63 mit seinem nicht dargestellten zylinderförmigen Ansatz eingeschraubt, um das Schraubenelement 61 zu bilden. Danach wird das so gebildete Schraubenelement 61 mit dem Gewindeschaft 62 voran durch die zweite Bohrung 68 in das Aufnahmeteil 66 eingeführt, bis der Schraubenkopf 63 am Rand der ersten Bohrung 67 anliegt. Anschließend wird das Druckelement 73 mit der sphärischen Ausnehmung voran durch die zweite Bohrung 68 in das Aufnahmeteil 66 eingeführt. Dann wird das Schraubenelement 61 in den Knochen bzw. Wirbel eingeschraubt. Schließlich wird der Stab 72 in das Aufnahmeteil 66 zwischen die beiden freien Schenkel 70, 70' eingelegt, die Winkelstellung des Aufnahmeteils relativ zu dem Schraubenelement justiert und mit der Innenschraube 71 fixiert. Durch das elastische Element werden Bewegungen um die Ruhelage in begrenzter Weise ermöglicht.

Wenn das elastische Element 1 wenigstens teilweise mit seinem durch die Schraubenfedern gebildeten elastischen Abschnitt über die Knochenoberfläche hervorsteht kann das Federelement 1 Biegekräfte, sowie Zug- und Druckkräfte aufnehmen. Steht das elastische Element mit seinem durch die Schraubenfedern gebildeten elastischen Abschnitt nicht mehr über die Knochenoberfläche hervor, kann das Schraubenelement 61 trotzdem bei einer Bewegung des Knochens bzw. Wirbels etwas nachgeben. Damit wird verhindert, dass ungünstige Spannungen auftreten.

Die Polyaxialschraube ist nicht auf die zuvor beschriebene Ausführungsform beschränkt, sondern kann auch jede beliebige andere Polyaxialschraube mit einem wie oben beschriebenen dreiteiligen Schraubenelement sein.

Die Stabfixierung ist nicht auf die in Fig. 8b gezeigte Innenschraube beschränkt, sondern es kann zusätzlich eine Außenmutter vorgesehen sein, oder jede bekannte Art der Stabfixierung kann eingesetzt werden.

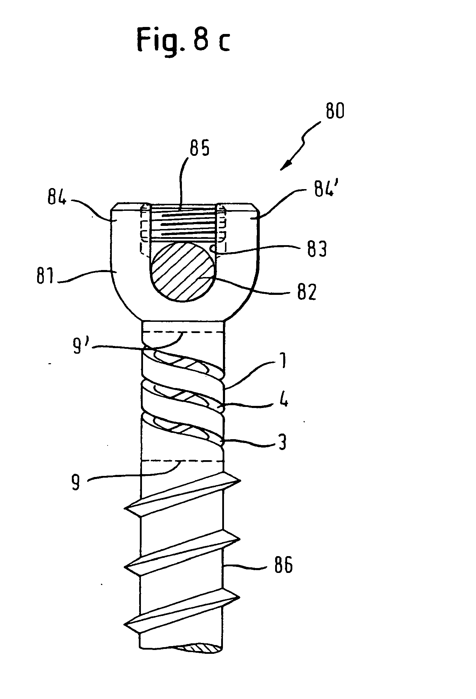

Als ein drittes Anwendungsbeispiel des erfindungsgemäßen elastischen Elements ist in Fig. 8c ein Knochenverankerungselement dargestellt, das als Monoaxialschraube 80 ausgebildet ist.

Bei der Monoaxialschraube 80 ist der Schraubenkopf als Aufnahmeteil 81 ausgebildet. Zur Aufnahme des Stabes 82 ist an das erste freie Ende des Aufnahmeteils 81 angrenzend eine U-förmige Ausnehmung 83 vorgesehen. Die durch die U-förmige Ausnehmung gebildeten freien Schenkel 84, 84' weisen auf ihrer Innenseite ein Innengewinde auf, in das das Aussengewinde einer Innenschraube 85 eingreift. Der Stab 82 wird im zusammengebauten Zustand der Monoaxialschraube zwischen dem Boden der U-förmigen Ausnehmung 83 und der Innenschraube 85 festgeklemmt. Angrenzend an ein dem ersten freien Ende gegenüberliegenden zweiten Ende des Aufnahmeteils 81 ist ein nicht dargestellter zylindrischer Ansatz mit einem Außengewinde vorgesehen, der in das an das erste Ende des elastischen Elementes 1 angrenzende Innengewinde 5 eingreift. Angrenzend an das dem ersten Ende des elastischen Abschnittes 1 gegenüberliegende .Ende weist die Monoaxialschraube einen Gewindeschaft 86 auf, der wie der oben beschriebene Gewindeschaft 62 der Polyaxialknochenschraube 60 ausgebildet ist und mit einem nicht dargestellten zylindrischen Ansatz mit einem Außengewinde in das Innengewinde 5' des elastischen Elements 1 eingreift.

Im Betrieb werden zunächst der Gewindeschaft 86 und das Aufnahmeteil in die beiden Innengewinde 5, 5' des elastischen Elements 1 eingeschraubt. Anschließend wird die Monoaxialschraube 80 in den Knochen oder den Wirbel eingeschraubt. Dann wird die U-förmige Ausnehmung 83 ausgerichtet und der Stab 82 eingelegt. Schließlich wird der Stab 82 mit der Innenschraube 85 fixiert.

Als weiteres Anwendungsbeispiel für das elastische Element 1 ist in Fig. 8d eine Draufsicht auf ein Verbindungselement 90 zu sehen, das aus einem stabförmigen Abschnitt 91, einem Federelement 1 und einer Platte 92 besteht.

Der stabförmige Abschnitt 91 weist einen nicht dargestellten zylindrischen Ansatz mit einem Außengewinde zum Einschrauben in das an das eine Ende des Federelementes 1 angrenzende Innengewinde 5 auf. Ebenso weist die Platte 92 einen nicht dargestellten zylindrischen Ansatz mit einem Außengewinde zum Einschrauben in das an das andere Ende des Federelementes 1 angrenzende Innengewinde 5' auf.

Die Platte besteht aus zwei in der Draufsicht kreisförmigen Abschnitten 93, 93', die über einen Stegabschnitt 94 miteinander verbunden sind. Die Breite B des Stegabschnittes 94 ist geringer als der Durchmesser D der kreisförmigen Abschnitte 93, 93'. Koaxial zu den kreisförmigen Abschnitten sind zwei Bohrungen 95, 95' für Senkschrauben durch die Platte vorgesehen.

Wie in Fig. 8d zu sehen weist die erste Seite 96 der Platte eine konvexe Krümmung auf während die zweite Seite 97 der Platte eine konkave Krümmung zum Anliegen dieser Seite an einen Knochen aufweist. Durch die unterschiedlichen Krümmungsradien der beiden Seiten 96, 97 der Platte 92 verjüngt sich die Platte 92 zu den seitlichen Rändern 98, 98' hin. Dadurch kann die Platte stabil und gleichzeitig raumsparend sein. Die Bohrungen 95, 95' sind in ihrer Form zur Aufnahme von Senkschrauben angepasst.

Abwandlungen der mit den Figuren 8a bis 8d beschriebenen Anwendungsbeispiele sind möglich. So wurde das stabförmige Element 50, die Polyaxialknochenschraube 60, die Monoaxialschraube 80 und das Verbindungselement 90 so beschrieben, dass das elastische Element 1 als separates Teil ausgebildet ist und mit den übrigen Teilen verschraubt ist. Es ist jedoch auch möglich, dass das erfindungsgemäße elastische Element als Abschnitt eines einstückigen stabförmigen Elementes, eines einstückigen Schraubenelementes, einer einstückigen Monoaxialschraube und eines einstückigen Verbindungselementes mit einem Stab- und einem Plattenabschnitt ausgebildet ist. Weiter ist es auch möglich das elastische Element mit Passsitz mit den anderen Elementen wie dem Gewindeschaft 62, dem Stababschnitt 51, 51', mit der Platte 92 oder dem Schraubenkopf 63 zu verbinden.

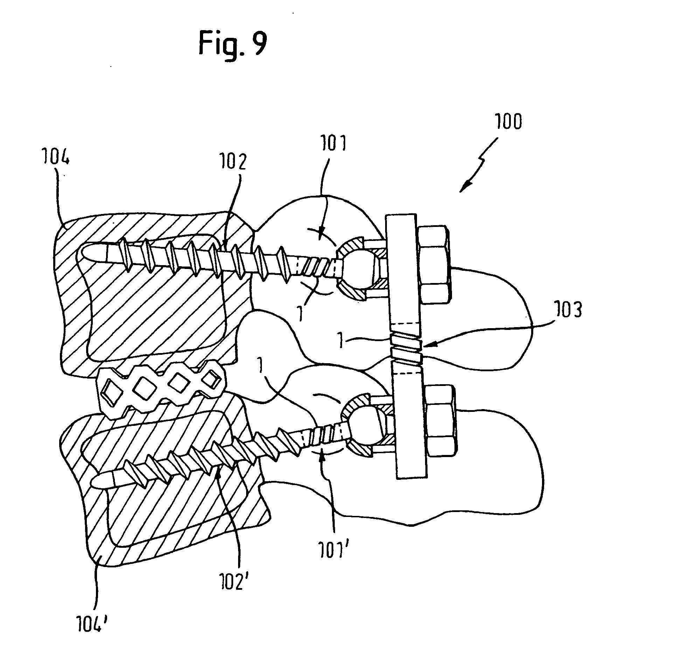

In Fig. 9 ist eine Stabilisierungsvorrichtung 100 für die Wirbelsäule dargestellt, wobei zwei Knochenverankerungselemente 101, 101' und ein diese verbindendes elastisches stabförmiges Element 103 vorgesehen ist. Die Schraubenelemente 102, 102' der Knochenverankerungselemente 101 bzw. 101' und das elastische stabförmige Element 103 weisen jeweils ein elastisches Element 1 auf. Die beiden Schraubenelemente 102, 102' sind in Wirbel 103, 103' eingeschraubt, sodass zwischen diesen Wirbeln über die Stabilisierungsvorrichtung 100 eine dynamische Stabilisierung hergestellt wird.

Durch die Mehrteiligkeit des elastischen stabförmigen Elementes und der Schraubenelemente ist es möglich, durch die Kombination von nur wenigen Grundelementen Stabilisierungsvorrichtungen 100 mit verschiedenen Eigenschaften zu erhalten. Die Stabilisierungsvorrichtung muß nicht notwendigerweise Knochenverankerungselemente mit einem elastischen Element und ein stabförmiges Element mit dem elastischen Element beinhalten. Je nach Anwendungsgebiet ist es auch möglich, nur ein stabförmiges Element mit einem elastischem Element und Knochenverankerungselemente ohne elastisches Element mit starren Schraubenelementen vorzusehen.

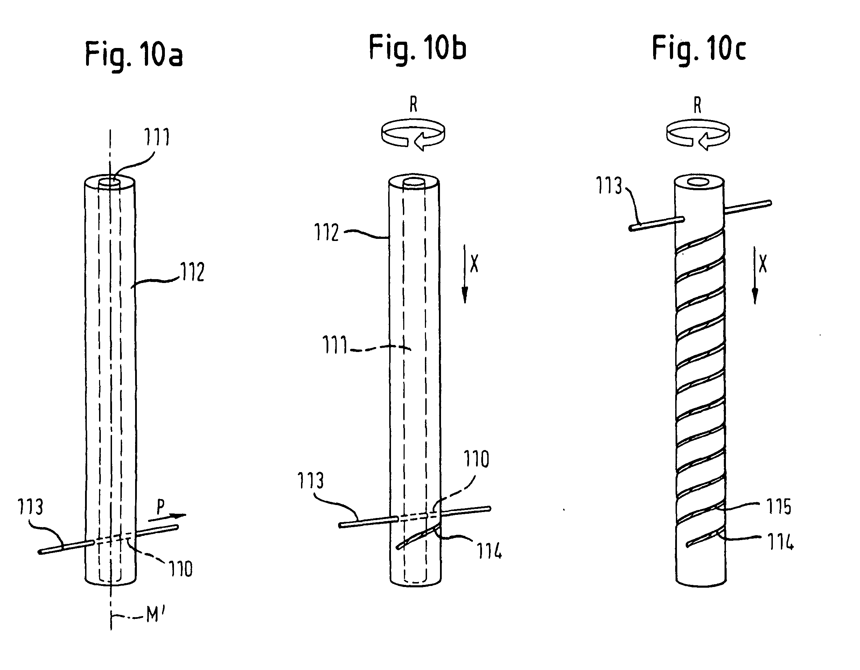

Die Herstellung eines elastischen Elementes 1 ist in den Figuren 10a, 10b, und 10c dargestellt. Dieses Herstellungsverfahren ist nicht Teil der Erfindung.

Zur Herstellung eines elastischen Elements 1 mittels Drahterodieren wird in einem Vollzylinder aus einem körperverträglichen Material, wie z. B. Titan, senkrecht durch die Mittenachse des Zylinders eine sich durch den gesamten Zylinder erstreckende erste Bohrung 110 erzeugt. Dann wird eine zweite Bohrung 111 koaxial zu der Mittenachse des Zylinders über dessen gesamte Länge gebildet, sodass ein Hohlzylinder 112 entsteht. Die Reihenfolge des Bildens der ersten und der zweiten Bohrung ist beliebig und kann auch umgekehrt sein. Anschließend wird durch die erste Bohrung 110 zum Drahterodieren ein Draht 113 durchgeführt. Fig. 10a deutet diesen Verfahrensschritt durch einen. Pfeil P an.

Im nächsten Schritt wird mit dem Draht 113 Drahterodieren durchgeführt, während der Hohlzylinder 112 entlang der Mittenachse in Richtung X mit konstanter Vorschubgeschwindigkeit relativ zum Draht verschoben und gleichzeitig mit konstanter Winkelgeschwindigkeit um seine Mittenachse gedreht wird. Die Drehung ist in Fig. 10b durch einen Pfeil D angedeutet. Dabei kommt es allein auf eine Relativbewegung zwischen Draht und Hohlzylinder an. So kann entweder der Draht raumfest sein und der Hohlzylinder bewegt werden oder aber auch umgekehrt. Auf diese Art und Weise werden durch Drahterodieren gleichzeitig zwei Schraubenlinien mit gleicher Steigung und zwei Ausnehmungen 114, 115 in der Wandung des Hohlzylinders gebildet, die in radialer Richtung in die Bohrung 111 münden. Fig. 10c zeigt das elastische Element kurz vor dem Ende des Schrittes des Drahterodierens. Nachdem die Ausnehmungen in axialer Richtung über eine vorbestimmte Länge gebildet worden sind, wird der Vorschub und die Drehung des Hohlzylinders gestoppt.

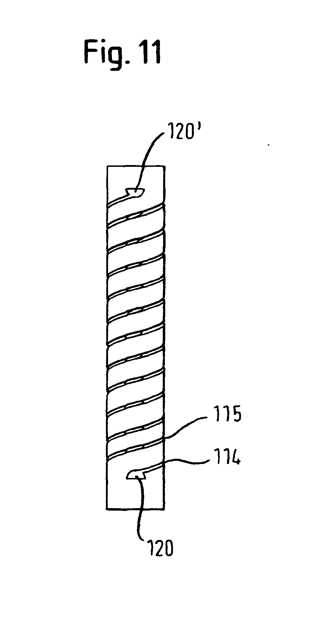

Wie in Fig. 11 dargestellt können zu Beginn des Drahterodierens und am Ende des Drahterodierens jeweils ein halbkreisförmiger Auslauf 120, 120' gebildet werde. Die Form des Auslaufs 120 bzw. 120' hat nicht zwingend die Form eines Halbkreises, sondern kann auch jede andere beliebige Form, wie die eines anderen Kreisabschnittes, haben, durch die Belastungsspitzen in dem Material am Übergang vom flexiblen Abschnitt zum festen Abschnitt im Betrieb gering gehalten werden.

Der Vorteil des oben beschriebenen Verfahrens mit Drahterodieren liegt für die Erzeugung des Auslaufs darin, dass die Ausläufe der beiden Schraubenfedern jeweils in einem gemeinsamen Arbeitsschritt hergestellt werden können, ein zusätzliches Umschalten zwischen den Achsen der Drahterodiermaschine ist bei dieser Ausführungsform im Vergleich zur Herstellung einer Einfachschraubenfeder nicht notwendig.

Schließlich wird in den zwei an die beiden Enden angrenzenden Endabschnitten der koaxialen Bohrung entlang der Mittenachse jeweils ein Innengewinde 5, 5' gebildet.

Alternativ kann das elastische Element 1 auch mittels Fräsen hergestellt werde. Dabei geht man wiederum von einem Zylinder mit einem vorbestimmten Außendurchmesser aus einem körperverträglichen Material, wie z.B. Titan, aus und fräst mit einem dünnen Scheibenfräser entlang einer ersten Schraubenlinie, deren Hauptachse kollinear zu der Hauptachse des Zylinders ist eine erste Ausnehmung. Dann wird in einem zweiten Schritt entlang einer zweiten Schraubenlinie, deren Windungen zwischen den Windungen der ersten Schraubenlinie verlaufen eine weitere Ausnehmung gebildet. Anschließend wird entlang der Hauptachse des Zylinders über die gesamte Länge des Zylinders eine Bohrung derart gebildet, dass die Ausnehmungen in diese Bohrung münden. Der Auslauf der Spirale am Übergang zwischen dem Spiralabschnitt und dem endseitigen Abschnitt des elastischen Elementes hat einen großen Einfluss auf die Stabilität des elastischen Elements 1. Daher wird mit einem Fingerfräser der Auslauf der Spirale an beiden Enden der Spirale derart nachbearbeitet, dass die scharfe Kante an der Innenseite der Bohrung entfernt wird. Dazu wird der Auslauf mit einem Fingerfräser in einem Winkel tangential zur Spiralkontur gefräst. Im Anschluss daran wird das Bauteil innen und außen entgratet.

Schließlich wird wie bei dem zuvor beschriebenen Verfahren in den zwei Endabschnitten der koaxialen Bohrung entlang der Mittenachse jeweils ein Innengewinde 5, 5' gebildet.

Weitere alternative Herstellungsverfahren für das elastische Element sind die Laserbearbeitung oder die Wasserstrahlbearbeitung, wobei die Herstellung analog dem Drahterodieren erfolgt, jedoch anstelle des gleichzeitigen Bildens von zwei Ausnehmungen durch einen durch die erste Bohrung durchgeführten Draht durch einen durch die erste Bohrung geführten Laserstrahl oder Wasserstrahl erfolgt.

In einem Verfahren gemäß der Erfindung werden anstelle der Innengewinde 5, 5' zu Beginn des Verfahrens durch Drehen zwei zylinderförmigen Ansätze mit je einem Außengewinde gebildet. In diesem Fall muss der Durchmesser der Bohrung 111 kleiner als der Durchmesser des zylinderförmigen Ansatzes sein.

Das Federelement wird ohne eine durchgehende Bohrung 111 hergestellt.

## Patentansprüche

1. Elastisches Element (20) zur Verwendung in einer Stabilisierungseinrichtung (100) für Knochen oder Wirbel,
welches als ein im wesentlichen zylinderförmiger Körper mit einem ersten Ende (22) und einem diesem gegenüberliegenden zweiten Ende (22') und mit einem elastischen Abschnitt zwischen dem ersten unddem zweiten Ende ausgebildet ist, wobei
der elastische Abschnitt aus wenigstens zwei Schraubenfedern (26, 27) ausgebildet ist, die koaxial derart angeordnet sind, dass die Windungen (3) einer Schraubenfeder (26) zumindest in einem Abschnitt der Schraubenfeder zwischen den Windungen (4) einer anderen Schraubenfeder (27) verlaufen, wobei
der elastische Abschnitt zwei in dem zylindrischen Körper ausgebildete Ausnehmungen (24, 25) umfasst, welche die wenigstens zwei Schraubenfedern (26, 27) ausbilden,
an das erste Ende angrenzend ein erster zylindrischer Ansatz (23) mit einem Außengewinde vorgesehen ist zum Verbinden mit einem Schaft (62) oder mit einem Kopf (63) einer Knochenschraube (61), zum Verbinden mit einem Stababschnitt (51) oder zum Verbinden mit einer Platte (92) und
an das zweite Ende (22') angrenzend ein zweiter zylindrischer Ansatz (23') vorgesehen ist, **dadurch** gekennzeichet dass der zweiten zylindrischen Ansatz mit einem Außengewinde vorgesehen ist zum Verbinden mit einem Schaft (62) oder mit einem Kopf (63) einer Knochenschraube (61), zum Verbinden mit einem Stababschnitt(51') oder zum Verbinden mit einer Platte (92), und dass
keine Bohrung koaxial zur Mittenachse M des elastischen Elements (20) vorgesehen ist.

2. Elastisches Element (20) nach Anspruch 1, wobei die Schraubenfedern (26, 27) den gleichen Außendurchmesser aufweisen.

3. Elastisches Element nach Anspruch 1 oder 2, wobei die Schraubenfedern (26, 27) in einer Ebene, die die Mittenachse der Schraubenfedern (26, 27) enthält, den gleichen Querschnitt der Windung (3,4) aufweisen.

4. Elastisches Element (20) nach einem der Ansprüche 1 bis 3 mit zwei im wesentlichen gleichen Schraubenfedern, wobei jede der beiden Schraubenfedern (26,27) relativ zur anderen um ihre gemeinsame Mittenachse um 180° gedreht ist.

5. Elastisches Element (20) nach einem der Ansprüche 1 bis 4, wobei
das elastische Element aus einem körperverträglichen Material, insbesondere Titan, besteht.

6. Elastisches Element (20) nach einem der Ansprüche 1 bis 5, bei dem der Außendurchmesser (D2) des elastischen Elements entlang der Mittenachse (M) variiert.

7. Elastisches Element (20) nach einem der Ansprüche 1 bis 6, bei dem die Steigung (α) der wenigstens zwei Schraubenfedern (31,32) entlang der Mittenachse (M) variiert.

8. Knochenverankerungselement (60,80,101,101') mit einem Schaft (62,86) zum Verankern im Knochen, wobei der Schaft einen Abschnitt mit einem elastischen Element (20) nach einem der Ansprüche 1 bis 7 aufweist, der einstückig oder mehrstückig ausgebildet ist.

9. Stabförmiges Element (50) zum Verbinden zweier
Knochenverankerungselemente (60,80,101,101') mit
einem elastischen Element (20) nach einem der Ansprüche 1 bis 7; und
einem starren Stababschnitt (51,51'), der an das eine Ende des elastischen Elements (20) angrenzt, wobei
das stabförmige Element entweder einstückig oder mehrstückig ausgebildet ist.

10. Stabilisierungseinrichtung (100) zur dynamischen Stabilisierung von Knochen, Knochenteilen oder der Wirbelsäule mit zumindest zwei Knochenverankerungselementen (101,101'), die über ein stabförmiges Element (103) oder eine Platte miteinander verbunden sind, wobei ein Abschnitt oder ein Element der Stabilisierungseinrichtung als ein elastisches Element (20) nach einem der Ansprüche 1 bis 8 ausgebildet ist.

11. Verfahren zur Herstellung des elastischen Elements (20) nach einem der Ansprüche 1 bis 7 mit den Schritten:
(a) Bereitstellen eines zylinderförmigen Körpers (112);
(b) Bilden einer ersten Ausnehmung (24) von außen in den zylinderförmigen Körper (112) entlang einer ersten Schraubenlinie;
(c) Bilden mindestens einer weiteren zweiten Ausnehmung (25) von außen in den zylinderförmigen Körper (112) entlang einer zweiten Schraubenlinie, wobei die Windungen der zweiten Schraubenlinie zumindest in einem Abschnitt zwischen den Windungen der ersten Schraubenlinie verlaufen so dass keine Bohrung koaxial zur Mittenachse M des elastischen Elements (20) entsteht und
(d) Bilden eines zylinderförmigen Ansatzes mit einem Außengewinde an den Endabschnitten des zylinderförmigen Körpers.

12. Verfahren nach Anspruch 11, wobei die Bildung der Ausnehmungen (24, 25) mittels Drahterodieren, Wasserstrahlbearbeitung, Laserstrahlbearbeitung oder einem spanabtragenden Verfahren erfolgt.

13. Verfahren nach Anspruch 11 oder 12, wobei die Erzeugung der ersten und der zweiten Ausnehmung (24, 25) in den Schritten (b) und (c) gleichzeitig erfolgt.

## Claims

1. Elastic element (20) for use in a stabilization device (100) for bones or vertebrae, which is formed as a substantially cylindrical body having a first end (22) and a second end (22') opposite the first end and having an elastic section between the first and the second end, wherein
the elastic section is formed by at least two helical springs (26, 27), which extend coaxially, such that the windings (3) of one helical spring (26) extend at least in a portion of the helical spring between the windings (4) of the other helical spring (27), wherein
the elastic section comprises two recesses (24, 25) formed in the cylindrical body, which form the at least two helical springs (26, 27),
adjacent the first end a first cylindrical boss (23) is provided having an outer thread for connection with a shaft (62) or with a head (63) of a bone screw (61), for connection with a rod section (51) or for connection with a plate (92), and
adjacent the second end (22') a second cylindrical boss (23') is provided, **characterized in that**
the second cylindrical boss (23') has an outer thread for connection with a shaft (62) or with a head (63) of a bone screw (61), for connection with a rod section (51') or for connection with a plate (92), and **in that**
no bore coaxial with the center axis M of the elastic element (20) is provided.

2. Elastic element according to claim 1, wherein the helical springs have a same outer diameter.

3. Elastic element according to claim 1 or 2, wherein the helical springs (26, 27) have a same cross section of the winding (3, 4) in a plane, which comprises the centre axis of the helical springs (26, 27).

4. Elastic element according to claims 1 to 3, having two substantially equal helical springs, wherein each of the two helical springs (26, 27) is rotated about the centre axis with respect to the other helical spring by 180 degrees.

5. Elastic element according to claims 1 to 4, wherein
the elastic element contains a body compatible material , in particular titanium.

6. Elastic element according to claims 1 to 5, wherein the outer diameter (D2) of the elastic element varies along the centre axis (M).

7. Elastic element according to claims 1 to 6, wherein the lead (α) of the at least two helical springs (31, 32) varies along the centre axis (M).

8. Bone anchoring element (60, 80, 101, 101') having a shaft (62, 86) for anchoring in the bone, wherein the shaft comprises a section including an elastic element (20) according to one of claims 1 through 7 formed as one single or multiple pieces.

9. Rod-shaped element (50) for connecting two bone anchoring elements (60, 80, 101, 101') having
an elastic element (20) according to one of claims 1 through 7; and
a rigid rod section (51, 51') adjacent to the one end of the elastic element (20), wherein the rod-shaped element is formed either as one single or as multiple pieces.

10. Stabilization device (100) for dynamic stabilization of bones, bone portions, or the vertebral column with at least two bone anchoring elements (101, 101'), which are connected to each other via a rod-shaped element (103) or a plate, wherein a section or an element of the stabilization device is formed as an elastic element (20) according to one of claims 1 through 8.

11. Method of manufacturing the elastic element (20) according to one of claims 1 through 7 including the steps:
(a) providing a cylindrical body (112);
(b) forming a first recess (24) from outside into the cylindrical body (112) along a first helical line;
(c) forming at least one further second recess (25) from outside into the cylindrical body (112) along a second helical line, wherein the windings of the second helical line extend at least in a section between the windings of the first helical line, such that no bore coaxial with the centre axis M of the elastic element (20) develops; and
(d) forming a cylindrical boss having an outer thread at the end sections of the cylindrical body.

12. Method according to claim 11, wherein the recesses are formed by wire eroding, waterjet treatment, laser treatment or a chip removing method.

13. Method according to claim 11 or 12, wherein the generation of the first and the second recess (24, 25) in steps (b) and (c) is performed simultaneously.

## Revendications

1. Élément élastique (20) à utiliser dans un dispositif de stabilisation (100) des os ou des vertèbres,
lequel est réalisé sous la forme d'un corps sensiblement cylindrique avec une première extrémité (22) et une seconde extrémité (22') opposée à celle-ci, et avec une partie élastique entre la première et la seconde extrémité,
la partie élastique étant formée par au moins deux ressorts hélicoïdaux (26, 27) agencés coaxialement, de telle sorte que les spires (3) d'un ressort hélicoïdal (26), au moins dans un tronçon du ressort, s'étendent entre les spires (4) d'un autre ressort hélicoïdal (27),
la partie élastique comprenant deux évidements (24, 25) réalisés dans le corps cylindrique et formant lesdits au moins deux ressort hélicoïdaux (26, 27),
une première protubérance (23) cylindrique avec un filetage extérieur, adjacente à la première extrémité, étant prévue pour être assemblée à une tige (62) ou à une tête (63) d'une vis pour os (61), pour être assemblée à un tronçon de tige (51) ou pour être assemblée à une plaque (92), et
une deuxième protubérance (23') cylindrique, adjacente à la seconde extrémité (22'), étant prévue,
**caractérisé**
**en ce que** la deuxième protubérance (23') cylindrique est prévue avec un filetage extérieur pour être assemblée à une tige (62) ou à une tête (63) d'une vis pour os (61), pour être assemblée à un tronçon de tige (51') ou pour être assemblée à une plaque (92), et
**en ce qu'**il n'est prévu aucune forure coaxiale à l'axe médian (M) de l'élément élastique (20).

2. Élément élastique (20) selon la revendication 1, dans lequel les ressorts hélicoïdaux (26, 27) ont le même diamètre extérieur.

3. Élément élastique selon la revendication 1 ou 2, dans lequel les ressorts hélicoïdaux (26, 27) possèdent la même section transversale pour la spire (3, 4) dans un plan contenant l'axe médian des ressorts hélicoïdaux (26, 27).

4. Élément élastique (20) selon l'une quelconque des revendications 1 à 3, comportant deux ressorts hélicoïdaux sensiblement identiques, chacun des deux ressorts hélicoïdaux (26, 27) étant tourné de 180° par rapport à l'autre ressort autour de leur axe médian commun.

5. Élément élastique (20) selon l'une quelconque des revendications 1 à 4, dans lequel l'élément élastique est réalisé dans un matériau biocompatible, en particulier du titane.

6. Élément élastique (20) selon l'une quelconque des revendications 1 à 5, dans lequel le diamètre extérieur (D2) de l'élément élastique varie le long de l'axe médian (M).

7. Élément élastique (20) selon l'une quelconque des revendications 1 à 6, dans lequel le pas (α) desdits au moins deux ressorts hélicoïdaux (31, 32) varie le long de l'axe médian (M).

8. Élément d'ancrage aux os (60, 80, 101, 101') comportant une tige (62, 86) pour l'ancrage dans l'os, ladite tige comportant une partie avec un élément élastique (20) selon l'une quelconque des revendications 1 à 7, lequel est réalisé d'une seule pièce ou en plusieurs pièces.

9. Élément en forme de tige (50) pour relier deux éléments d'ancrage aux os (60, 80, 101, 101'), comportant
un élément élastique (20) selon l'une quelconque des revendications 1 à 7 ; et
une partie de tige (51, 51') rigide qui est adjacente à une des extrémités de l'élément élastique (20),
ledit élément en forme de tige étant réalisé d'une seule pièce ou en plusieurs pièces.

10. Dispositif de stabilisation (100) pour la stabilisation dynamique des os, des parties d'os ou de la colonne vertébrale, comportant au moins deux éléments d'ancrage aux os (101, 101'), qui sont reliés entre eux par l'intermédiaire d'un élément en forme de tige (103) ou d'une plaque, une partie ou un élément du dispositif de stabilisation étant réalisé sous la forme d'un élément élastique (20) selon l'une quelconque des revendications 1 à 8.

11. Procédé pour la réalisation d'un élément élastique (20) selon l'une quelconque des revendications 1 à 7, comportant les étapes :
(a) mise à disposition d'un corps cylindrique (112) ;
(b) réalisation d'un premier évidement (24) depuis l'extérieur dans le corps (112) cylindrique le long d'une première ligne hélicoïdale ;
(c) réalisation d'au moins un deuxième évidement (25) supplémentaire depuis l'extérieur dans le corps (112) cylindrique le long d'une deuxième ligne hélicoïdale, les spires de la deuxième ligne hélicoïdale s'étendant au moins dans un tronçon entre les spires de la première ligne hélicoïdale, de telle sorte qu'il ne se forme aucune forure coaxialement à l'axe médian (M) de l'élément élastique (20) ; et
(d) formation d'une protubérance cylindrique avec un filetage extérieur sur les zones d'extrémité du corps cylindrique.

12. Procédé selon la revendication 11, selon lequel les évidements (24, 25) sont ménagés par électroérosion par fil, par usinage au jet d'eau, par usinage au rayon laser ou par un procédé travaillant par enlèvement de copeaux.

13. Procédé selon la revendication 11 ou 12, selon lequel le premier et le deuxième évidement (24, 25) sont réalisés simultanément au cours des étapes (b) et (c).
